(19) **Europäisches Patentamt**

**European Patent Office**

**Office européen des brevets**

(11) **EP 1 337 228 B1**

(12) **EUROPEAN PATENT SPECIFICATION**

(45) Date of publication and mention
of the grant of the patent:
**07.12.2005 Bulletin 2005/49**

(51) Int Cl.[7]: **A61K 38/43**, A61K 33/10

(21) Application number: **02744528.7**

(86) International application number:
**PCT/US2002/019756**

(22) Date of filing: **21.06.2002**

(87) International publication number:
**WO 2003/003976 (16.01.2003 Gazette 2003/03)**

(54) **COMPOSITIONS FOR REMOVING HUMAN CERUMEN**

ZUSAMMENSETZUNGEN ZUR ENTFERNUNG VON MENSCHLICHEM CERUMEN

COMPOSITIONS PERMETTANT D'ENLEVER LE CERUMEN HUMAIN

(84) Designated Contracting States:
**AT BE CH CY DE DK ES FI FR GB GR IE IT LI LU
MC NL PT SE TR**

(30) Priority: **03.07.2001 US 302959 P**

(43) Date of publication of application:
**27.08.2003 Bulletin 2003/35**

(73) Proprietor: **Alcon, Inc.
6331 Hünenberg (CH)**

(72) Inventors:
• **CAGLE, Gerald, D.
Fort Worth, TX 76116 (US)**
• **OWEN, Geoffrey, R.
Southlake, TX 76092 (US)**
• **RIDRUEJO, Nuria, Jimenez
E-08019 Barcelona (ES)**
• **WALL, G., Michael
Fort Worth, TX 76109 (US)**

(74) Representative: **Best, Michael et al
Lederer & Keller
Patentanwälte
Prinzregentenstrasse 16
80538 München (DE)**

(56) References cited:
GB-A- 1 161 192    US-A- 4 904 469
US-A- 5 954 682    US-A- 6 093 417

**Description**

Field of the Invention

The present invention generally pertains to the removal of human cerumen. More particularly, but not by way of limitation, the present invention is directed to advantageous compositions for assisting in the removal of human cerumen.

Description of the Related Art

Cerumen, or ear wax, is a mixture of secretions from the ceruminous and pilosebaceous glands as well as squamae of epithelium, dust, and other debris. Cerumen forms a protective layer on the skin of the external ear canal. The consistency of, and thus the difficulty in removing, cerumen varies from individual to individual and is at least partially genetically determined.

Cerumen build-up and impaction in the external ear canal is a significant problem, especially for the infant and geriatric populations of the world. In the United States about 8 million cerumen removals take place each year, and in the United Kingdom the number is 2 million. Individuals possessing hairy ear canals, narrow ear canals, or osteoma are more disposed to such build-up or impaction. In addition, some literature suggests that the use of cotton buds to clean the external ear canal interferes with the body's normal shedding of ear wax and epithelium and increases the chance of such build-up and impaction. Build-up and/or impaction of ear wax may cause irritation, itching, pain, infection, or conductive hearing loss. Wax removal is necessary to alleviate these conditions. Cerumen removal is also required when it is necessary to examine the tympanic membrane.

Various compositions for softening or removing human cerumen are known. Several products contain carbamide peroxide (6.5%) in an anhydrous glycerin vehicle as defined in the FDA monograph part 344. These include products such as Murine® Ear Drops available from Abbott Laboratories of Columbus, Ohio; Debrox® Drops Earwax Removal Aid available from SmithKline Beecham of Pittsburgh, Pennsylvania; Bausch & Lomb Earwax Remover available from Bausch & Lomb of Rochester, New York; and Flents® Earwax Remover available from Flents Products Co. of Yonkers, New York. Another commercially available product is Cerumenex® Eardrops, which is a prescription product containing triethanolamine polypeptide oleate-condensate (10%) available from the Purdue Frederick Company of Norwalk, Connecticut. Cerumenex® sometimes results in irritation of the ear canal.

In addition, the literature speaks of certain other agents that are known to be somewhat effective in softening ear wax. Such agents include glycerin (glycerol), olive oil, almond oil, mineral oil, sodium carbonate, sodium bicarbonate, hydrogen peroxide, docusate sodium, and dichlorobenzene. After softening with one of these agents, irrigation with body temperature water or saline is often performed to remove the softened cerumen. Dichlorobenzene sometimes results in irritation of the ear canal.

Compositions that facilitate the removal of ear wax have also been the subject of several patents. For example, U.S. Patent No. 3,422,186 (Sasmor) discloses cerumenolytic compositions comprising ethylene oxide-polyoxypropylene glycol condensates; U.S. Patent No. 4,895,875 (Winston) discloses stabilized peroxide solutions comprising urea peroxide and glycerin and methods of preparation and use useful for cerumen removal; U.S. Patent No. 5,296,472 (Sanchez et al.) discloses compositions comprising cyclodextrins and methods of use for cerumen removal; U.S. Patent No. 5,380,711 (Sanchez et al.) discloses oil-free "empty" cyclodextrin compositions and methods of use for cerumen removal; and U.S. Patent No. 5,480,658 (Melman) discloses compositions comprising acetic acid and boric acid in a water base useful for cleaning the external ear canal of pets.

An aqueous solution of five percent sodium bicarbonate is often made by physicians and used to effectively treat impacted cerumen. This solution may be prepared with or without glycerin. However, these solutions are not stable, and for this reason, sodium bicarbonate solutions have never been developed into commercial products. If they are used, physicians are inconvenienced by preparing the solution for each individual patient.

Despite the above-described cerumenolytic agents and compositions, a need exists for an improved composition for assisting in the removal of human cerumen that is commercially viable and that does not suffer from the limitations of currently available cerumenolytics. The present invention provides advantageous compositions to meet this need.

Summary of the Invention

One aspect of the present invention is a composition for assisting in the removal of human cerumen that includes bicarbonate and a cerumenolytically acceptable enzyme. The composition preferably also includes an aqueous otologically acceptable vehicle that acts as a carrier. The compositions of the present invention are commercially viable and provide a safe and effective means of removing human cerumen from the external ear canal.

Brief Description of the Drawings

For a more complete understanding of the present invention, and for further objects and advantages thereof, reference is made to the following description taken in conjunction with the accompanying drawings in which:

FIG. 1 is an exploded view of a preferred device suitable for the delivery of certain preferred compositions of the present invention;
FIG. 2 is a cross-sectional view of FIG. 1;
FIG. 3 is a front view of the device of FIG. 1 in the assembled state in which the first and second parts of the composition are not mixed;
FIG. 4 is a cross-sectional view of FIG. 3;
FIG. 5 is an enlarged, cross-sectional view of the upper container of the device of FIG. 1;
FIG. 6 is a sectional view of FIG. 5 taken along line 6-6;
FIG. 7 is a top view of the safety ring of the device of FIG 1;
FIG. 8 is an exploded, cross-sectional, fragmentary view of the tubular sleeve, upper container, and cap of the device of FIG. 1;
FIG. 9 is a front view of the device of FIG. 1 in the assembled state in which the first and second parts of the composition have been mixed, the cap is removed, and the device is ready to dispense the mixed composition; and
FIG. 10 is a cross-sectional, fragmentary view of FIG. 9.

Detailed Description of the Invention

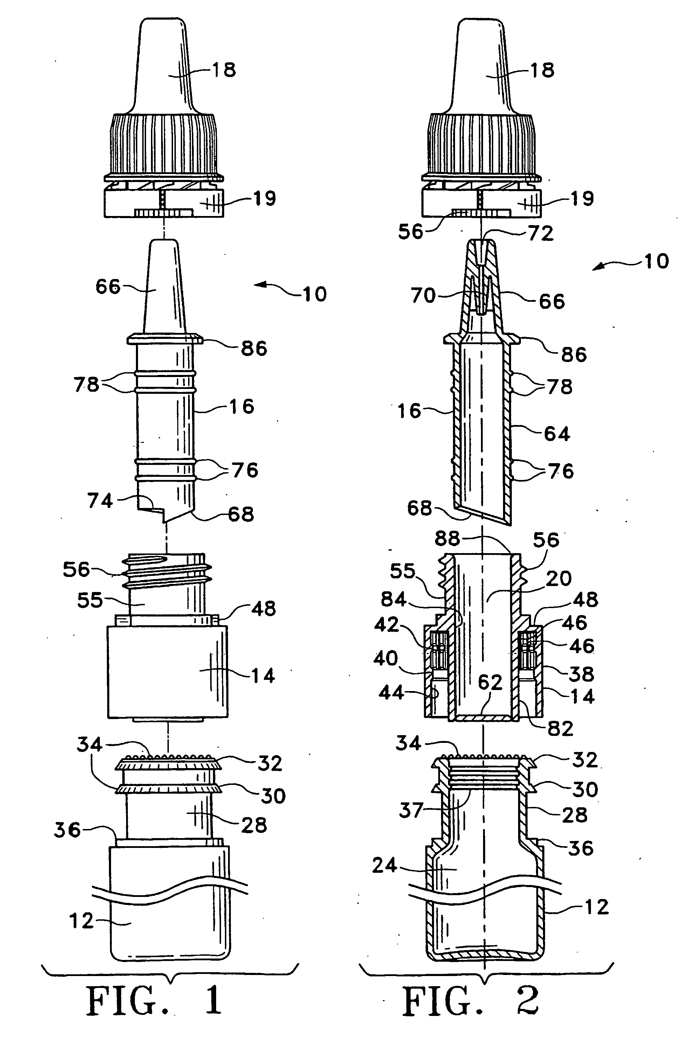

The preferred embodiments of the present invention and their advantages are best understood by referring to FIGS. 1-10 of the drawings, like numerals being used for like and corresponding parts of the various drawings.

Unless otherwise indicated, all ingredient concentrations listed as a percentage are in units of weight/volume percent.

As used herein, the term "bicarbonate" refers to any soluble bicarbonate salt. These salts are most frequently formed with group I metals. Sodium bicarbonate, potassium bicarbonate, or mixtures thereof are the preferred bicarbonates.

As used herein, the term "otologically acceptable vehicle" refers to any substance or combination of substances that act as a carrier for an active agent or agents and that are suitable for administration to the external ear canal. By way of example, an otologically acceptable vehicle may comprise any combination of preservatives, surfactants, viscosity enhancers, penetration enhancers, buffers, sodium chloride or other salts, solubilizers, stabilizers, pH adjusters, tonicity agents, fillers, demulcents, and water. The otologically acceptable vehicle for the compositions of the present invention is preferably an aqueous vehicle.

The preferred preservatives for the compositions of the present invention include, but are not limited to, poly [dimethylimino-2-butene-1,4-diyl]chloride-alpha-[4-tris(2-hydroxyethyl)ammonium]dichloride, which is available from Onyx Chemical Corporation as Polyquarternium 1 or Onamer M™, or from Alcon Laboratories, Inc. as Polyquad®; benzalkonium halides such as benzalkonium chloride; alexidine salts; chlorhexidine salts; hexamethylene biguanimides and their polymers; and mixtures thereof.

The surfactant of the present invention preferably is nonionic, and may include, but is not limited to, polysorbates, such as polysorbate 20 available from ICI Americas Inc. of Wilmington, Delaware under the trademark Tween® 20; 4-(1, 1, 3, 3-tetramethylbutyl) phenol/poly(oxyethylene) polymers, such as the polymer sold under the trademark Tyloxapol; poly(oxyethylene)-poly(oxypropylene) block copolymers; polyethylene glycol esters of fatty acids, such as coconut, polysorbate, polyoxyethylene, and polyoxypropylene ethers of higher alkanes ($C_{12}$ -$C_{18}$). Examples of the preferred class include polysorbate 20 polyoxyethylene (23) lauryl ether (Brij® 35), polyoxyethylene (40) stearate (Myrj® 52), polyoxyethylene (25) propylene glycol stearate (Atlas® G2612). Brij® 35, Myrj® 52 and Atlas® G 2612 are trademarks of, and are commercially available from, ICI Americas Inc. Most preferably the nonionic surfactant is selected from poly(oxyethylene)-poly(oxypropylene) block copolymers and mixtures thereof. Such surfactant components can be obtained commercially from the BASF - Corporation under the trademarks Pluronic® and Tetronic®. Such block copolymers can be generally described as polyoxyethylene/polyoxypropylene condensation polymers terminated in primary hydroxyl groups. They may be synthesized by first creating a hydrophobe of desired molecular weight by the controlled addition of propylene oxide to the two hydroxyl groups of propylene glycol. In the second step of the synthesis, ethylene oxide is added to sandwich this hydrophobe between hydrophile groups.

Preferred buffers of the present invention include, but are not limited to, citrate, phosphate, borate, acetate, Tris, and their salts and mixtures thereof. Bicarbonates may also act as a buffer for the compositions of the present invention. Other conventional buffer components may also be employed to maintain or adjust the pH of the composition.

The compositions of the present invention employ an enzyme, as will be described in greater detail hereinbelow. The enzyme may comprise a solid composition, such as a powder or tablet, or a liquid composition. The liquid enzyme compositions of the present invention preferably comprise an enzyme, stabilizing agents, and water. Enzyme stabilizing agents preferred for use with the compositions of the present invention include, but are not limited to, monomeric polyols, polymeric polyols, calcium ions, and borate/boric acid compound.

As used herein, the term "monomeric polyol" refers to a compound with 2 to 6 carbon atoms and at least two hydroxy groups. Examples of monomeric polyols are glycerin, propylene glycol, ethylene glycol, sorbitol and mannitol. Preferably, the monomeric polyols are selected from polyols having 2-3 carbons and at least two hydroxy groups ("2-3 carbon polyol"). Examples of 2-3 carbon polyols are glycerin, 1,2-propane diol ("propylene glycol"), 1,3-propane diol and ethylene glycol. Glycerin and propylene glycol are the most preferred 2-3 carbon polyols.

As used herein, the term "polymeric polyol" refers to a polyalkoxylated glycol with a molecular weight ranging from about 200-600 Daltons. Examples of polymeric polyols are polyethylene glycol 200 (denoting a molecular weight of 200 Daltons, "PEG 200") and PEG 400. The PEGs may optionally be monoalkoxylated. Examples of monoalkoxylated PEGs are monomethoxy PEG 200 and ethoxy PEG 400. Though these alkoxylated PEGs are not technically polyols, they are similar in structure to the non-alkoxylated PEGs; therefore, for defining purposes, they are included in the term "polymeric polyol."

If a combination of monomeric and polymeric polyols is used, the amounts of monomeric polyol and polymeric polyol will vary depending on the particular combination of polyols used. In general, such liquid enzyme compositions will require about 40 to 85% weight/volume ("% w/v") of a polyol mixture to achieve the necessary criteria for efficacious and commercially viable liquid enzyme compositions, as described above. The ratio of monomeric to polymeric polyols is also important. In general, the monomeric polyol:polymeric polyol ratio will be from 1:5 to 5:1, with a preferred ratio being 2:1 to 1:2, weight:weight. While any of the polyols can be components of the compositions of the present invention, particular polyols may be used depending on the particular intended use. For example, propylene glycol, which has preservative activity, is a preferred monomeric polyol when the need for an additional preservative present in a liquid enzyme composition of the present invention is desired. The most preferred combination of polyols used in the compositions of the present invention are glycerin and PEG-400. The most preferred amount of the glycerin/PEG-400 combination is 25% w/v glycerin with 50% w/v PEG-400.

The liquid enzyme compositions of the present invention may also contain an effective amount of calcium ion. The calcium ion contained in the compositions of the present invention may be obtained by the addition of various calcium salts. For example, the calcium ion source may be obtained from calcium chloride, calcium acetate and calcium ascorbate or other water soluble salts of calcium. The most preferred calcium ion source is calcium chloride. As used herein, "effective amount of calcium ion" refers to that amount of calcium ion which enhances the proteolytic stability of an enzyme in the liquid enzyme compositions of the present invention. While that amount will vary depending upon the various components present, typical calcium ion concentrations will be about 1 to 90 millimolar. Preferred concentrations will be about 4.5 to 45 millimolar, and most preferred concentrations will be of from 10 to 25 millimolar.

The liquid enzyme compositions of the present invention may also contain an effective amount of a borate/boric acid compound. As used herein, "borate/boric acid compound" refers to an inorganic compound comprising boron and one or more oxygen groups, and which is either in acid or base form when dissolved in a composition of the present invention. Sources of borate/boric acid compounds include alkali metal salts of borate, boric acid and borax. As used herein, "effective amount of a borate/boric acid compound" refers to that amount of a borate/boric acid compound contained in a liquid enzyme composition of the present invention which enhances the proteolytic stability of the enzyme. While such an amount will vary depending on other components present, the amount will be about 0.3 to 8.0% (w/v). Preferred amounts will be of from 0.5 to 2.0% (w/v). The borate/boric acid compound may also contribute to the anti-microbial preservation of the liquid enzyme compositions of the present invention to a level effective for multi-use dispensing. The solubility of the borate/boric acid compound may be limited in water. The solubility of these compounds, however, may be increased by increasing the amount of polyol employed.

Preferred demulcents for the compositions of the present invention include, but are not limited to, povidone, polyvinyl alcohol, glycerin, propylene glycol, polyethlene glycol, and cellulose derivatives such as hydroxypropyl methyl cellulose (HPMC).

A first composition which is described here as reference includes bicarbonate and an aqueous otologically acceptable vehicle. Sodium bicarbonate is the preferred bicarbonate. Sodium bicarbonate is preferably present in the amount of about 0.5 % to about 15 %. The amount of sodium bicarbonate is most preferably about 5 %. The aqueous otologically acceptable preferably includes a demulcent, a surfactant, a preservative, and a buffer, or combinations of any of the foregoing. Glycerin is a preferred demulcent, and it is preferably present in the amount of about 1 % to about 20 %. Sodium citrate.2$H_2$O is a preferred buffer, and it is preferably present in the amount of about 0.1 % to about 8 %. Tetronic® 1304 is a preferred surfactant, and it is preferably present in the amount of about 0.05 % to about 1 %. Benzalkonium chloride is a preferred preservative, and it is preferably present in the amount of about 0.001 % to about 0.1 %. Buffers are-preferably present in a quantity sufficient to adjust the pH of the composition from about 7.5 to about 9.0.

Examples 1 and 2 hereinbelow provide in vitro data showing that the first composition is efficacious in assisting in the removal of human and artificial cerumen. The first composition is preferably packaged in a bottle having a syringe or dropper to dispense the composition, or in a plastic bottle that may be squeezed to dispense the composition.

To administer the composition, a user first tilts his or her head toward one shoulder or assumes a reclined position on his or her side. The user then substantially fills the external ear canal with the composition. Approximately 1 to 2 ml of the composition typically fills the external ear canal. The user keeps his or her head titled, or remains in such a reclined position, for a sufficient time period for the composition to bathe the external ear canal and to dislodge, break-up, and/or digest cerumen in the ear canal. This time period is preferably from about fifteen minutes to about thirty minutes, although in certain cases the time period may be shorter or longer. Unlike many known cerumenolytic agents, it is believed that the aqueous compositions of the present invention may not require irrigation with water or saline to complete cerumen removal due to its efficacy in dislodging, breaking-up, and digesting cerumen. Alternatively, following the termination of the bathing period, irrigation with body temperature water, saline, or other rinsing fluid may be performed to facilitate removal of cerumen from the ear canal.

A second composition which is described here as reference includes an enzyme and an otologically acceptable vehicle. The otologically acceptable vehicle is preferably identical, or substantially similar, to the otologically acceptable vehicle described above in connection with the first composition. However, the otologically acceptable vehicle of the second composition may also include an enzyme stabilizing agent. Glycerin is a preferred enzyme stabilizing agent.

The enzymes that may be utilized in the compositions and methods of the present invention include all enzymes that are useful in softening, dislodging, breaking-up, and/or digesting human cerumen in the external ear canal and cause, at most, only minor irritation to the external ear canal. For purposes of this specification, enzymes that satisfy the foregoing requirements are referred to as being "cerumenolytically acceptable". It has been discovered that preferred cerumenolytically acceptable enzymes include lipases, proteases, and amylases. In addition, combinations of lipases and proteases, lipases and amylases, and proteases and amylases may be used. The most preferred enzymes are proteases, which are also referred to herein as proteolytic enzymes.

Examples of cerumenolytically acceptable proteolytic enzymes which may be utilized in the present invention include but are not limited to pancreatin, trypsin, subtilisin, collagenase, keratinase, carboxypeptidase, papain, bromelain, aminopeptidase, elastase, Aspergillo peptidase, pronase E (from S. griseus), dispase (from Bacillus polymyxa) and mixtures thereof. If papain is used, a reducing agent, such as N-acetylcysteine, may be required. Microbially derived enzymes, such as those derived from Bacillus, Streptomyces, and Aspergillus microorganisms, represent another cerumenolytically acceptable preferred type of enzyme which may be utilized in the present invention. Of this sub-group of enzymes, the most preferred are the Bacillus derived alkaline proteases generically called "subtilisin" enzymes.

The identification, separation and purification of enzymes is known in the art. Many identification and isolation techniques exist in the general scientific literature for the isolation of enzymes, including those enzymes having proteolytic and mixed proteolytic/lipolytic/amylolytic activity. The enzymes contemplated by this invention can be readily obtained by known techniques from plant, animal or microbial sources. In addition, with the advent of recombinant DNA techniques, it is anticipated that new sources and types of stable proteolytic enzymes will become available. Such enzymes should be considered to fall within the scope of this invention so long as they meet the criteria set forth herein.

Pancreatin, subtilisin and trypsin are preferred enzymes for use in the present invention. Pancreatin is extracted from mammalian pancreas, and is commercially available from various sources, including Scientific Protein Laboratories of Waunakee, Wisconsin; Novo Nordisk of Denmark; Sigma Chemical Co. of St. Louis, Missouri; and Boehringer Mannheim of Indianapolis, Indiana. Pancreatin USP is a mixture of proteases, lipases and amylases, and is defined by the United States Pharmacopeia ("USP"). The most preferred form of pancreatin is Pancreatin 9X. As utilized herein, the term "Pancreatin 9X" means a filtered (0.2 microns) pancreatin containing nine times the USP protease unit content. Subtilisin is derived from Bacillus bacteria and is commercially available from various commercial sources including Novo Industries; Fluka Biochemika of Buchs, Switzerland; and Boehringer Mannheim. Trypsin is a 23,800 dalton protease with 6 disulfide bridges. Trypsin can be synthesized or obtained from various sources, such as porcine, bovine or swine pancreatin. Trypsin is also available from commercial sources such as Sigma Chemical Co., Biofac Co. of the United Kingdom; and Novo Nordisk. Trypsin may vary from species to species, but in general will be highly homologous with porcine or human trypsin.

The most preferred enzymes of the present invention are the alkyl trypsins ("Al-trypsin(s)"). Al-trypsins are more stable in the liquid compositions than the native trypsin, or other native enzymes. As used herein, "Al-trypsin" refers to a covalently modified trypsin wherein one or more of its lysine epsilon-amino groups has been monoalkylated or di-alkylated to form the corresponding monoalkylamino or dialkylamino group. The alkyl group attached to the amine may be a primary or branched $C_{1-12}$ group. Preferred Al-trypsins of the present invention are those wherein the alkyl group is a primary or branched $C_{1-4}$ group. Alkylation of trypsin is generally performed by reductive alkylation. The degree of alkylation of the lysine epsilon-amino groups will depend on the reaction conditions of the reductive alkylation process. For example, if the reaction cycle is repeated a number of times and/or a higher reagent to enzyme ratio is used, then full alkylation, i.e., alkylation of all of the lysine epsilon-amino groups, will tend to be achieved. Al-trypsins of the present invention will preferably be greater than about 80 percent dialkylyated at all of their lysine epsilon-amino groups.

The most preferred Al-trypsin is methyl trypsin ("Me-trypsin"). The most preferred Me-trypsin of the present invention will be derived from porcine tissue sources and will be greater than about 80 percent dimethylated, as described above. Methyl trypsin is preferably present in the amount of about 50 AU/ml to about 500 AU/ml. Methyl trypsin is most preferably present in the amount of 200 AU/ml. For purposes of this specification, an "activity unit" or "AU" is defined as the amount of enzyme activity necessary to generate one microgram (mcg) of tyrosine per minute ("mcg Tyr/min"), as determined by the casein-digestion, colorimetric assay described below. 200 AU/ml is equivalent to about 600 USP units of trypsin/ml, and corresponds to about 0.25 mg of methyl trypsin per ml.

**Casein-digestion assay**

A 5.0 mL portion of casein substrate (0.65% casein w/v) is equilibrated for 10 minutes (min)+/- 0.5 seconds (sec) at 37° C. A 1.0 mL portion of enzyme solution (0.2 mg/ml) is then added to the casein substrate and the mixture vortexed, then incubated for 10 min+/- 0.5 sec at 37°C. After incubation, 5.0 mL of 14% trichloroacetic acid is added and the resultant mixture immediately vortexed. The mixture is incubated for at least another 30 min, then vortexed and centrifuged for 15-20 min (approx. 2000 rpm). The supernatant of the centrifuged sample is filtered into a serum filter sampler and a 2.0 mL aliquot removed. To the 2.0 mL sample is added 5.0 mL of 5.3% sodium carbonate solution. The sample is vortexed, 1.0 mL of 0.67 N Folin's Phenol reagent is added, and the sample is immediately vortexed again, then incubated for 60 min at 37°C. The sample is then read on a visible light spectrophotometer at 660 nanometers versus purified water as the reference. The sample concentration is then determined by comparison to a tyrosine standard curve.

Al-trypsins may be synthesized by the process of reductive alkylation of trypsin, as is more fully described in U.S. Patent No. 6,228,323, which is incorporated herein in its entirety by this reference. Me-trypsin is also available from commercial sources such as Sigma Chemical Co. and Promega Corp. (Madison, Wis.).

During storage, some of the activity of the enzyme may be lost, depending on length of storage and temperature conditions. Thus, the enzymes of the compositions of the present invention may be prepared with initial amounts of enzyme that exceed the concentration ranges described herein. The preferred compositions of the present invention will generally contain one or more enzymes in an amount of about 50-1000 AU/ml in the combined solution. The compositions will most preferably contain about 100-500 AU/ml in the combined solution, which corresponds to pancreatin in the range of about 0.1 to 2% w/v; subtilisin in a range of about 0.01 to 0.2% w/v; trypsin in the range of about 0.01 to 0.2% w/v; and methyl trypsin in the range of about 0.01 to 0.2% w/v.

Examples 1 and 2 hereinbelow provide in vitro data showing the second composition is efficacious in assisting in the removal of human and artificial cerumen.

Because many cerumenolytically acceptable enzymes are unstable in highly aqueous solutions after a period of days or weeks, the second composition is preferably prepared and packaged as two separate parts that are mixed prior to administration to the external ear canal. The first part preferably comprises the cerumenolytically acceptable enzyme and the enzyme stabilizer of the otologically acceptable vehicle, and the second part preferably comprises the remainder of the otologically acceptable vehicle. Reference Example 3 hereinbelow describes the first part and the second part of an exemplary second composition of the present invention, as well as a preferred method of making said first and second parts.

Once made; the first and second parts may be removed from their separate packages and mixed. A bottle with a syringe or dropper, or a squeezable plastic dispenser bottle, is preferably used to deliver the mixed composition to the ear as described hereinabove in connection with the first composition.

Alternatively, the second composition may be packaged in a single device having separate containers or compartments for the first part and the second part. Such a device preferably enables the mixing of the two parts prior to administration, and also enables the administration of the mixed composition to the external ear canal as described hereinabove in connection with the first composition. FIGS. 1-10 illustrate such a device 10 suitable for the packaging of the first part 22 and the second part 26 of the second composition according to a preferred embodiment.

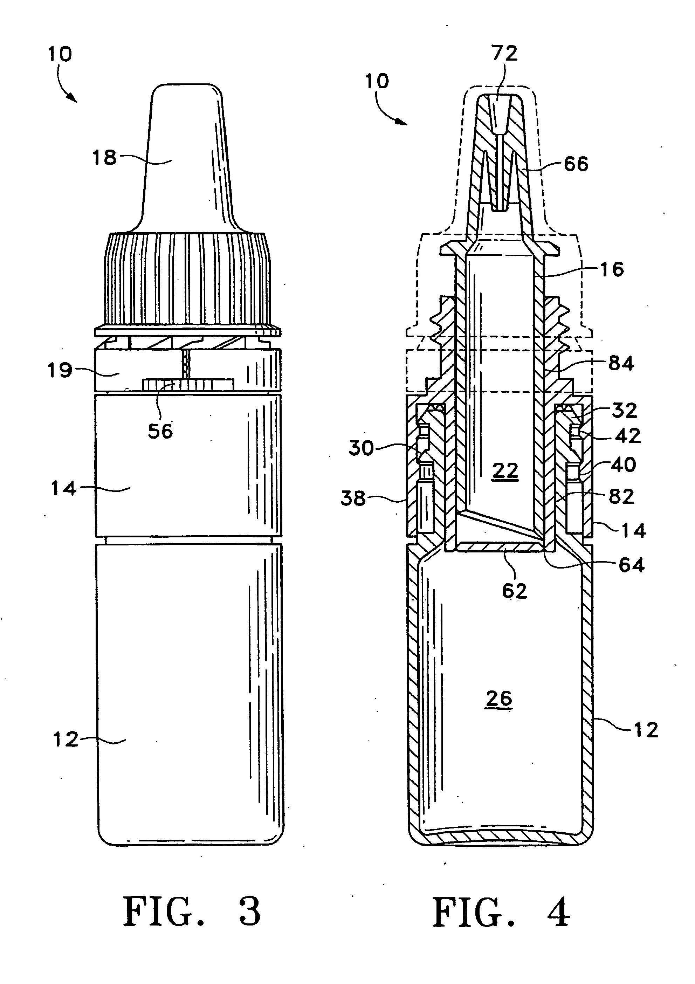

As shown best in the exploded views of FIGS. 1-2, device 10 generally includes a lower container 12, and upper container 14, a tubular sleeve or member 16, a cap 18, and a safety seal 19. As shown best in FIGS. 3 and 4, in the assembled state of device 10, upper container 14 is coupled to lower container 12, tubular sleeve 16 is disposed within and coupled to upper container 14, cap 18 covers tubular sleeve 16 and is removably coupled to upper container 14, and safety seal 19 is disposed around upper container 14 below cap 18. Cap 18 and safety seal 19 are shown in dashed lines in FIG. 4 for clarity of illustration. The various portions of device 10 are preferably formed from conventional polymeric materials. Most preferably, lower container 12 is formed of low density polyethylene, upper container 14 is formed of high density polyethylene, tubular sleeve 16 is formed of Zylar, a copolymer available from NOVA Chemicals of Leominster, Massachusetts, and cap 18 is formed of polypropylene. Upper container 14 has a reservoir 20 for holding the first part 22. Lower container 12 has a reservoir 24 for holding the second part 26.

Lower container 12 includes a hollow neck 28 that includes two ring shaped edges 30 and 32. Stria 34 are located on edges 30 and 32. A shoulder 36 is located at the junction of neck 28 and reservoir 24. Sealing rings 37 are located on the internal surface of neck 28 above shoulder 36.

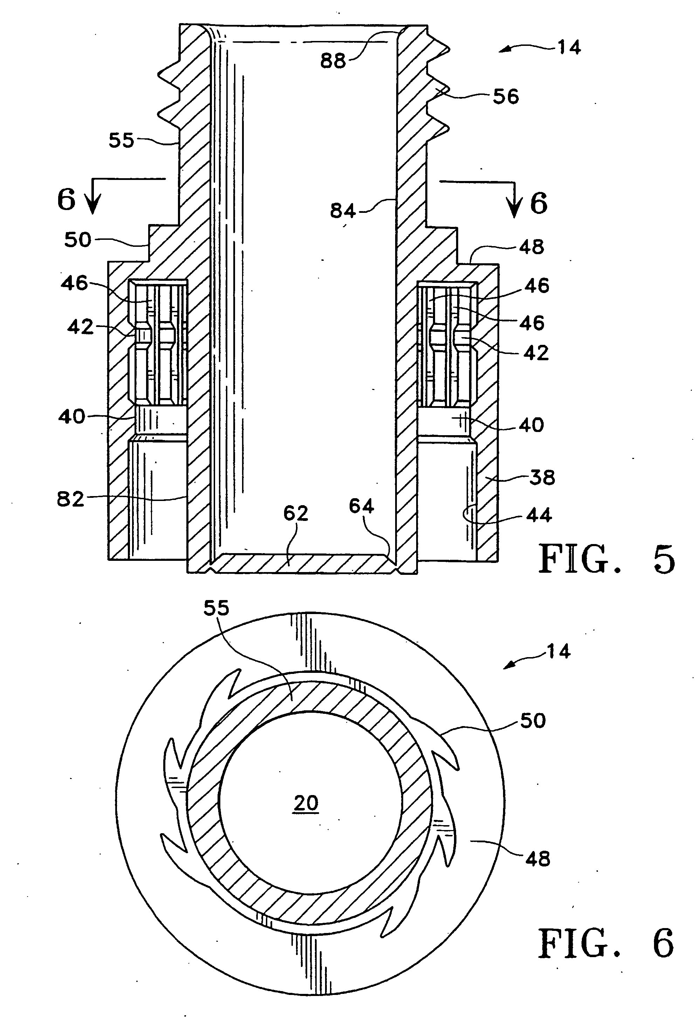

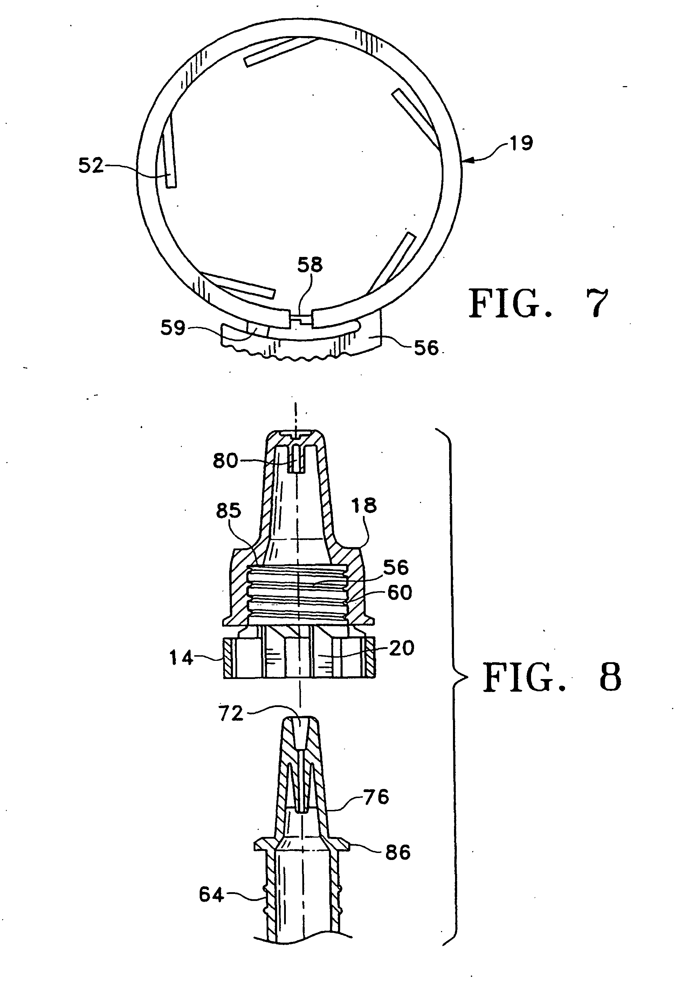

As shown best in FIGS. 2 and 4, upper container 14 includes a surface 82 that mates with sealing rings 37 of lower container 12 to prevent leakage of second part 26 from reservoir 24 or entry of air into reservoir 24. Upper container 14 also includes a flap 38 that surrounds neck 28 of lower container 12. As shown best in FIG. 5, flap 38 includes two ring shaped ribs 40 and 42 disposed on the internal surface 44 of flap 38 and for mating with edges 30 and 32 of lower container 12. Stria 46 run vertically along internal surface 44 from rib 40 to the internal surface of shoulder 48. Although not shown in the FIGS., stria 46 also run horizontally and radially along the internal surface of shoulder 48 toward the longitudinal axis of upper container 14. When upper container 14 is disposed on lower container 12, stria 34 of edges 30 and 32 couple with stria 46 to prevent upper container 14 and lower container 12 from rotating relative to each other. As shown best in FIGS. 5-6, a plurality of sawteeth 50 are disposed on the external surface of shoulder 48. As shown in FIG. 7, sawteeth 50 mate with flexible wings 52 to removably couple safety seal 19 to upper container 14. Safety seal 19 has a ring-shaped geometry with a perforation at connecting point 58. Safety seal 19 has an axial wing 56 that is connected to the remainder of seal 19 at a connecting point 59. Upper container 14 also has a neck 55 having external threads 56 for mating with internal threads 60 of cap 18, as is best shown in FIG. 8. As is best shown in FIG. 5, upper container 14 has bottom or non-permeable membrane 62 that is tearable along a perforated line 64 disposed about the periphery of bottom 62.

Tubular sleeve 16 includes a hollow body 64 having a truncated cone-shaped portion 66 on one end and a helicoidal edge 68 on an opposite end. Truncated cone-shaped portion 66 has a internal channel 70 terminating in a reverse truncated cone-shaped hole 72 that serves as the nozzle or dropper to dispense the mixed composition from device 10. Alternatively truncated cone-shaped portion 66 may be modified to include a conventional "Luer Lok" that complies with Luer Taper Specification 70.1 of the American Standards Association and that allows coupling to a syringe, cannula, or other conventional medical instruments. Helicoidal edge 68 preferably has a small horizontal section 74. Tubular sleeve 16 also includes a first sealing ring 76 and a second sealing ring 78 disposed on the external surface of body 64. Sealing rings 76 and 78 mate with internal surface 84 of reservoir 20 of upper container 14 to prevent leakage of first part 22 from reservoir 20 or entry of air into reservoir 20. Sealing ring 78 is especially useful in preventing such leakage or entry when first part 22 is a liquid. As shown in FIG. 8, cap 18 preferably has a member 80 that seals hole 72 when cap 18 is screwed onto upper container 14.

The above-referenced description is a summary of the structure of device 10. Certain portions of device 10 are described in greater detail in U.S. Patent Nos. 5,474,209 and 5,782,345, which are incorporated herein by reference.

Referring generally to FIGS. 1-10, the preferred use of device 10 to mix the first part 22 and second part 26 of the second composition and to administer the mixed composition to the external ear canal will be described in greater detail. As shown in FIGS. 3-4, device 10 is in a first position in which the first part 22 and the second part 26 are unmixed. To mix parts 22 and 26, a user first moves axial wing 56 of safety seal 19 radially outward, breaking connecting point 59. The user then holds axial wing 56 and rotates safety seal 19 about a longitudinal axis of device 10, causing sawteeth 50 of upper container 14 to engage flexible wings 52 of safety seal 19. Such rotation splits safety seal 19 at connection point 58. The user can then remove safety seal 19 from device 10.

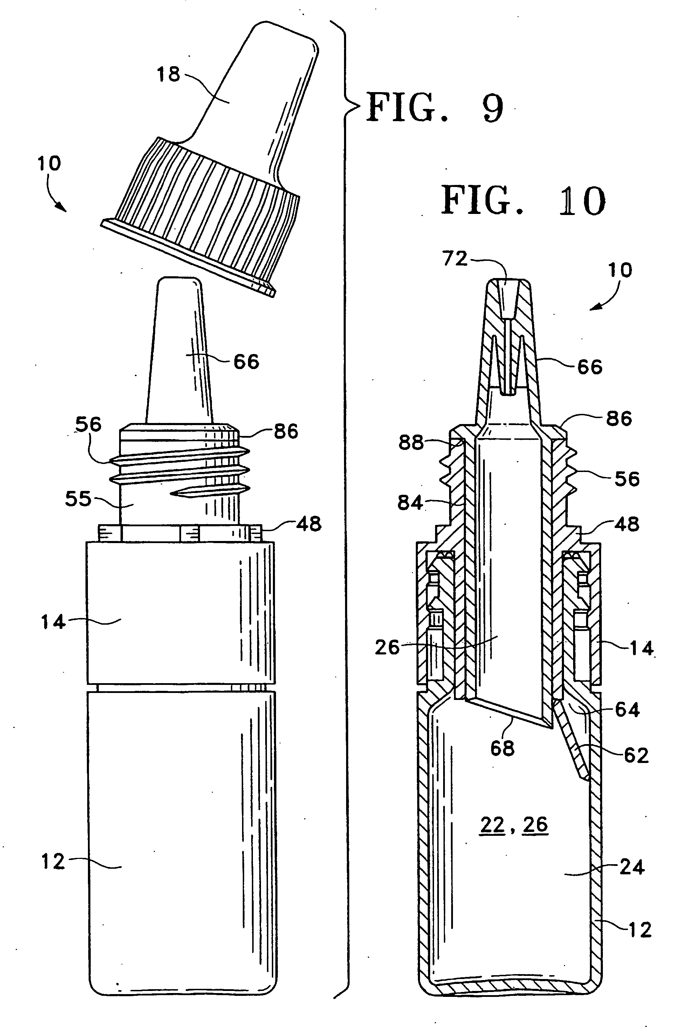

Next, the user screws cap 18 downward onto neck 55 of upper container 14. During this downward travel of cap 18, internal shoulder 85 of cap 18 contacts external shoulder 86 of tubular sleeve 16. Tubular sleeve is pushed downward within upper container 14 until external shoulder 86 of tubular sleeve 16 contacts shoulder 88 of upper container 14. Referring to FIG. 10, as tubular sleeve 16 is pushed downward, helicoidal edge 68 tears bottom 62 of reservoir 20 along perforation 64, with the exception of a portion of perforation 64 at horizontal section 74 of edge 68. Bottom 62 is thus opened but remains connected to upper container 14. First part 22 is then in communication with second part 26, and may be further mixed by shaking device 10, if necessary. In this second position of device 10 in which parts 22 and 26 are mixed, sealing rings 76 and 78 mate with internal surface 84 of upper container 14 to prevent leakage of the first or second parts of the composition from reservoirs 20 and 24 or entry of air into the reservoirs. Upon removal of cap 18, the mixed composition may be dispensed into the external ear canal by squeezing on the external surface of lower container 12 so as to force the mixture through nozzle 66 of tubular sleeve 16. When cap 18 is re-threaded onto tubular sleeve 16, member 80 seals hole 72.

A third composition which is the composition of the present invention includes bicarbonate, a cerumenolytically acceptable enzyme, and an otologically acceptable vehicle. Sodium bicarbonate is the preferred bicarbonate. Sodium bicarbonate is preferably present in the amount of about 0.5% to about 15%. The amount of sodium bicarbonate is most preferably about 5%. The cerumenolytically acceptable enzyme for the third composition is preferably identical to those described above in connection with the second composition. The cerumenolytically acceptable enzyme is preferably present in the amount of about 50 AU/ml to about 1000 AU/ml. The most preferred cerumenolytically acceptable enzymes are proteolytic enzymes. The proteolytic enzyme is preferably present in the amount of about 50 AU/ml to about 1000 AU/ml. The most preferred proteolytic enzyme is methyl trypsin. Methyl trypsin is preferably present in the amount of about 50 AU/ml to about 500 AU/ml. Methyl trypsin is most preferably present in the amount of 200 AU/ml. The otologically acceptable vehicle is preferably identical, or substantially similar, to the otologically acceptable vehicle described above in connection with the second composition. Examples 1, 2, 4, 5, and 7 hereinbelow provide in vitro data showing that the third preferred composition is efficacious in assisting in the removal of human and artificial cerumen.

Similar to the second composition, the third composition is preferably prepared and packaged as two separate parts, which are mixed prior to administration to the external ear canal. The first part preferably comprises the cerumenolytically acceptable enzyme and the enzyme stabilizer of the otologically acceptable vehicle, and the second part preferably comprises the sodium bicarbonate and the remainder of the otologically acceptable vehicle. Examples 6, 8, 9, and 10 hereinbelow describe examples of the first part and the second part of the third composition of the present invention, as well as a preferred method of making said first and second parts.

Once made, the first and second parts may be removed from their separate packages and mixed. A bottle with a syringe or dropper, or a squeezable plastic dispenser bottle, is preferably used be used to deliver the mixed composition to ear as described hereinabove in connection with the first preferred composition. Alternatively, the third composition may be packaged in and administered to the external ear canal via a single device having separate containers or compartments for the first part and the second part. A preferred device is device 10 described above in connection with the second preferred composition.

As described hereinabove, all the compositions of the present invention may be packaged in a bottle with a syringe or dropper to dispense the composition, a plastic bottle that may be squeezed to dispense the composition, or a device such as device 10. It is preferred that the bottled compositions also be packaged in a blister pouch or pack made from aluminum foil in order to minimize any unwanted rise in the pH of the composition. When so packaged, such compositions, unlike currently known aqueous sodium bicarbonate based cerumenolytics, are pH stable at up to 40 degrees Celsius for up to 3 months after preparation.

In all of the compositions of the present invention that are packaged in a plastic bottle or device and that use benzalkonium chloride as a preservative, the otologically acceptable vehicle preferably also includes sodium citrate. $2H_2O$ or another citrate. Such bottles and devices are preferably made from a soft plastic, such as polyethylene, and are preferably sterilized via conventional ethylene oxide (ETO) methods. It has been unexpectedly discovered that citrate helps to maintain the concentration of benzalkonium chloride over time and thus yields a product that is more commercially viable.

All of the compositions of the present invention that comprise a liquid enzyme are preferably prepared in a sterile manner by filtration through an appropriate microbial filter. After the first and second parts of the compositions have been sterilized by filtration, a further advantage of the device 10 is that the first and second parts of the composition are admixed aseptically. This is because device 10 containing the first and second parts of the compositions is assembled in an aseptic manner with an air-tight seal, thus sterile mixing is performed within the closed sterile system of the device.

The following examples are intended to illustrate, but in no way limit, the present invention.

## EXAMPLE 1

Table 1 shows examples of the preferred compositions of the present invention, the compositions of certain components of these exemplary compositions, and the vehicles for these exemplary compositions. All ingredient amounts are in units of weight/volume percent unless otherwise indicated. Composition A is an example of the first composition, composition B is an example of the second composition after the mixing of its first and second parts, and compositions C and D are examples of the third composition after the mixing of their first and second parts, respectively. Composition A is also the "bicarbonate component of Composition C", and Composition B is also the "proteolytic enzyme component of Composition C". Composition $D_1$ is the "bicarbonate component of Composition D", and Composition $D_2$ is the "proteolytic enzyme component of Composition D". $V_1$ is the otologically acceptable vehicle for Compositions A, B, and C, and $V_2$ is the otologically acceptable vehicle for Composition D. Sodium chloride was added to $V_2$ and $D_2$ so that these compositions would have an equivalent salt content as $V_1$.

**TABLE 1**

| Ingredient | A | B | C | D | $V_1$ | $V_2$ | $D_1$ | $D_2$ |
|---|---|---|---|---|---|---|---|---|
| Sodium Bicarbonate | 5 | --- | 5 | 5 | --- | --- | 5 | --- |
| Sodium Citrate. $2H_2O$ | 3 | 3 | 3 | --- | 3 | --- | --- | --- |
| Sodium Chloride | --- | --- | --- | --- | --- | 3.48 | --- | 3.48 |
| Methyl Trypsin | --- | 200 AU/ml | 200 AU/ml | 200 AU/ml | --- | --- | --- | 200 AU/ml |
| Glycerin | 7 | 7 | 7 | 7 | 7 | 7 | 7 | 7 |
| Tetronic® 1304 | 0.25 | 0.25 | 0.25 | --- | 0.25 | --- | --- | --- |
| Benzalkonium Chloride | 0.01 | 0.01 | 0.01 | 0.01 | 0.01 | 0.01 | 0.01 | 0.01 |
| pH | 8.1 | 8.3 | 8.3 | 8.0 | 8.3 | 6.0 | 7.9 | 7.0 |
| Water | q.s. | q.s. | q.s. | q.s. | q.s. | q.s. | q.s. | q.s. |

EP 1 337 228 B1

**EXAMPLE 2**

Compositions A and B were tested for their efficacy in assisting in the removal of cerumen in the following manner.

Samples of human cerumen were provided by various otology clinics. Samples were variable in type and amount. Therefore, about 20 to 30 specimens were pooled and mixed in a pestle and mortar to form a larger, relatively homogeneous sample lot. This enabled a series of assays to be carried out with a single lot of human cerumen.

In order to overcome the lot-to-lot variation in human cerumen, an artificial cerumen was developed. The artificial cerumen consists of a mixture of three components. The first component (50%) is a lipid mixture based upon the reported composition of the lipids in ear wax. The second component (30%) is homogenized bovine corneal epithelial cells, which simulates the desquamated epidermal cells in ear wax. The third component (20%) is lyophilized fetal bovine serum, which simulates the other components of ear wax that are secreted from the ceruminous and sebaceous glands.

**Preparation of 14 grams of Artificial Cerumen**

1. The following lipids are weighed out into a glass vessel, and then dissolved in 70 mls of chloroform/methanol (2:1 v/v) with warming:

| Lipid Ingredient | Vendor | Amount (grams) |
|---|---|---|
| Squalene | ICN Biochemicals, Inc. of Auora, Ohio ("ICN") | 0.448 |
| Cholesterol | Research Chemical Ltd., Heysham, Lancaster, England | 1.463 |
| Cholesterol Palmitate | ICN | 0.336 |
| Cholesterol Stearate | Sigma Chemical Co. | 0.336 |
| Oleic Acid OleylEster | Sigma Chemical Co. | 0.326 |
| Oleic Acid Stearyl Ester | Sigma Chemical Co. | 0.326 |
| Triolein | ICN | 0.105 |
| Oleic Acid | Mallinckrodt Baker Inc., of Paris, Kentucky | 2.097 |
| Stearic Acid | Calbiochem Biosciences, Inc. of LaJolla, California | 0.795 |
| Cholesterol Sulfate | Sigma Chemical Co. | 0.140 |
| Phosphatidylcholine, dioleoyl | Sigma Chemical Co. | 0.131 |
| Phosphatidylcholine, distearoyl | Sigma Chemical Co. | 0.131 |
| Phosphatidylcholine, dipalmitoyl | Sigma Chemical Co. | 0.131 |
| Phosphatidylcholine, dimyristoyl | Sigma Chemical Co. | 0.131 |
|  |  |  |
| Total |  | 6.896 |
|  |  |  |
| Triglyceride concentrate Chicken egg yolk (suspension) 43.39mg/ml | Calbiochem Biosciences, Inc. of LaJolla, California | 0.242 mls (0.105 grams)* |
| Total |  | 7.001 |

* = The triglyceride suspension is mixed with water in step 4.

2. The bovine corneal epithelial cells were prepared as follows. Frozen bovine mature eyes (total of 305) were purchased from Pel-Freeze®. After thawing in water, the corneal epithelial cells were scraped from the eyes using a razor blade, and accumulated in 200 mls of deionized water. This suspension was treated with a Polytron® homogenizer using a setting of 4 for 1 minute. The resulting suspension was then lyophilized to give 4.2 grams of dried bovine corneal epithelial cells.

3. The dried fetal bovine serum was prepared as follows. Fetal bovine serum (100 ml) was purchased from Hyclone and lyophilized to give 5.34 grams of a pale pink powder. A portion of this powder (2.8 grams) was used in the preparation of artificial cerumen.

4. The artificial cerumen was prepared as follows. Using a 5 inch glass mortar and pestle, the corneal epithelial cells (4.2 grams) and dried fetal bovine serum (2.8 grams) were mixed with a few drops of water to form a paste. To this paste was added the chloroform/methanol solution of the lipids (fume hood) and the mixture was ground with the mortar to produce a uniform paste. This was allowed to slowly evaporate in the fume hood until the odor of chloroform had disappeared. The artificial wax was then collected and stored in a sealed and air-tight container in a refrigerator (4°C). Before use in the efficacy testing, the artificial cerumen was warmed to 37°C.

**In Vitro Efficacy Testing (Human and Artificial Cerumen)**

About 30mg samples of the pooled lot of human cerumen or the artificial cerumen were accurately weighed using an analytical balance. Each sample was then gently rolled into a ball using the fingers (gloved), and placed in 12x75 mm borosilicate glass test tubes. Each test solution was pre-warmed and then added (2ml) to a cerumen sample in a water-bath set at 37°C, where it was allowed to stand without agitation.

At the time interval (30 minutes or 2 hours), the contents of each test tube were pipetted into separate syringes pre-fitted with Acrodisc® filters. Using finger pressure, the sample was filtered into size 13 (100mm) borosilicate glass test-tubes. Some samples are difficult to filter completely, and so filtration attempts are stopped after 90 seconds, and the analysis then proceeds with the volume collected. This procedure is repeated in triplicate for each test solution. Staggering the digestions for 5 or 10 minutes can conveniently achieve this.

Absorbencies at 600nm were recorded usually without any dilution of the filtrate. In the event that the absorbency was above 0.7 units, or that there was less than 1 ml of filtrate, appropriate dilutions were made. Absorbencies at 280nm were made on diluted solutions of the filtrate. A suitable dilution is usually 0.2 ml of the filtrate combined with 4 ml of water (factor of 21). The absorbency of each original test solution was also recorded at both wavelengths using the same dilutions as the corresponding test sample.

The cerumenolytic activity of the test solution is defined as the absorbance units released by the test solution from a gram of cerumen per ml of solution, after the incubation. For calculations, the absorbance reading of the solution blank with an identical dilution to the test sample solution was subtracted from the sample reading, and this net absorbance was then multiplied by the dilution factor and the 2 ml test volume to obtain the total units. The total absorbance units were then divided by the weight, in grams, of the cerumen sample, as follows:

((Absorbency of test solution - aborbency of solution blank) x 2 x dilution factor) / sample

weight in grams = absorbency/gram/ml

The absorbency at 280nm indicates the amount of protein digested by the composition, while the absorbency at 600nm indicates the amount of color, lipids and other ingredients released from the cerumen.

**Results**

Table 2 represents the amount of cerumen digested by each composition as measured by a spectrophotometer in absorbency units per gram of cerumen per ml of composition alter the cerumen has been immersed in the composition for 2 hours. The 280 nanometer wavelength is referred to herein as the "protein component". The 600 nanometer wavelength is referred to herein as the "lipid component". As shown by Table 2, Compositions A and B were each effective in digesting the protein component and the lipid component of both the human and artificial cerumen.

## TABLE 2

### Efficacy Results
### (Absorbency Units Per Gram Per ml at 2 Hours)

| Cerumen | A | B | C | $V_1$ | $A - V_1$ | $B - V_1$ | $(A - V_1) + (B - V_1)$ | $C - V_1$ |
|---|---|---|---|---|---|---|---|---|
| Human 280 nm | 1659 | 1654 | 1740 | 1120 | + 539 | + 534 | + 1073 | + 620 |
| Human 600 nm | 8.0 | 10.6 | 9 | 11.2 | - 3.2 | - 0.6 | - 3.8 | - 2.2 |
| Artificial 280 nm | 365 | 166 | 854 | 166 | + 199 | 0 | + 199 | + 688 |
| Artificial 600 nm | 18.8 | 6.4 | 52 | 4.6 | + 14.2 | + 1.8 | + 16. | + 47.4 |

### Reference EXAMPLE 3

Table 3 shows the composition of the first part of Composition B before mixing with the second part of Composition B. Table 4 shows the composition of the second part of Composition B before mixing with the first part of Composition B. Composition B is preferably made by simple mixing of the composition of Table 3 with the composition of Table 4. All ingredient amounts are in units of weight/volume percent unless otherwise indicated.

TABLE 3

| (First Part of Composition B) | |
|---|---|
| **INGREDIENT** | **AMOUNT** |
| Glycerin | 46.7 |
| Methyl Trypsin | 1335 AU/ml |
| Purified water | q.s. |

TABLE 4

| (Second Part of Composition B) | |
|---|---|
| **INGREDIENT** | **AMOUNT** |
| Tetronic® 1304 | 0.294 |
| Benzalkonium Chloride | 0.012 |
| Sodium Citrate.$2H_2O$ | 3.529 |
| Citric Acid | q.s. to pH 8.0 |
| Purified water | q.s. |

Various volumes of the first part and the second part of Composition B may be employed. The preferred ratio of the volume of the first part of Composition B to the volume of the second part of Composition B is 3:17. A preferred volume of the first part is 1.5 ml. A preferred volume of the second part is 8.5 ml. The glycerin in the first part of Composition B acts as an enzyme stabilizing agent for the methyl trypsin. After the first part and the second part of Composition B are mixed, the glycerin also acts as a demulcent.

### EXAMPLE 4

Composition C was tested for its efficacy in assisting in the removal of human cerumen in the manner described in Example 2. Table 2 represents the amount of cerumen digested by Composition C as measured by a spectrophotometer in absorbency units per gram of cerumen per ml of composition after the cerumen as been immersed in the composition for 2 hours. As shown by Table 2, Composition C was effective in digesting the protein component and the lipid component of both the human and artificial cerumen.

In addition, it was unexpectedly discovered that composition C exhibits a synergistic effect in digesting artificial cerumen. Referring again to Table 2, the absorbency measurements for the bicarbonate component A of Composition C, the proteolytic enzyme component B of Composition C, and the vehicle $V_1$ of Composition C are also provided. As shown by Table 2, the sum of the absorbency measurements of the bicarbonate component A and the proteolytic enzyme component B, subtracting any contribution from vehicle $V_1$, is significantly less than the absorbency measurement for Composition C, subtracting any contribution from vehicle $V_1$, for both the protein component and the lipid component of artificial cerumen.

**EXAMPLE 5**

Composition C was also tested for its efficacy in removing cerumen as compared to Murine® Ear Drops and Cerumenex® Eardrops. Composition C, Murine, and Cerumenex were tested in the manner described in Example 2, except that absorbency values were measured at 30 minutes instead of 2 hours.

Table 5 shows the amount of cerumen digested by Composition C, Cerumenex, and Murine as measured by a spectrophotometer in absorbency units per gram of cerumen per ml of composition after the cerumen has been immersed in the composition for 30 minutes. As shown by Table 5, Composition C was much more effective in removing the protein component of both human and artificial cerumen than either Murine or Cerumenex. Composition C was also much more effective in removing the lipid component of artificial cerumen than either Murine or Cerumenex. In addition, as shown by Table 5 and Table 8 (discussed hereinbelow), Composition C was much more effective in removing the protein and lipid components of artificial cerumen than a five percent sodium bicarbonate solution.

## TABLE 5

### Efficacy Results
### (Absorbency Units Per Gram Per ml at 30 Minutes)

| Cerumen | C | Murine® | Cerumenex® |
|---|---|---|---|
| Human 280 nm | 970 | 53 | 133 |
| Human 600 nm | 8 | 14 | 9 |
| Artificial 280 nm | 628 | 27 | 65 |
| Artificial 600 nm | 85 | 0 | 3 |

**EXAMPLE 6**

Table 6 shows the composition of the first part of Composition C before mixing with the second part of Composition C. Table 7 shows the composition of the second part of Composition C before mixing with the first part of Composition C. Composition C is preferably made by simple mixing of the composition of Table 6 with the composition of Table 7. All ingredient amounts are in units of weight/volume percent unless otherwise indicated.

## TABLE 6
### (First Part of Composition C)

| INGREDIENT | AMOUNT |
|---|---|
| Glycerin | 46.7 |
| Methyl Trypsin | 1335 AU/ml |
| Purified water | q.s. |

## TABLE 7
### (Second Part of Composition C)

| INGREDIENT | AMOUNT |
|---|---|
| Tetronic® 1304 | 0.29 |
| Sodium Bicarbonate | 5.9 |
| Benzalkonium Chloride | 0.012 |
| Sodium Citrate.2H$_2$O | 3.53 |
| Citric Acid | q.s. pH 8.0 |
| Purified water | q.s. |

Various volumes of the first part and the second part of Composition C may be employed. The preferred ratio of the volume of the first part of Composition C to the volume of the second part of Composition C is 3:17. A preferred volume of the first part is 1.5 ml. A preferred volume of the second part is 8.5 ml. The glycerin in the first part of Composition C acts as an enzyme stabilizing agent for the methyl trypsin. After the first part and the second part of Composition C are mixed, the glycerin also acts as a demulcent.

### EXAMPLE 7

Composition D was tested for its efficacy in assisting in the removal of cerumen as compared to an aqueous solution of five percent sodium bicarbonate. Composition D and the five percent sodium bicarbonate solution were tested in the manner described in Example 2, except that absorbency values were measured at 30 minutes instead of 2 hours.

Table 8 shows the amount of cerumen digested by Composition D and the five percent sodium bicarbonate solution as measured by a spectrophotometer in absorbency units per gram of cerumen per ml of composition after the cerumen has been immersed in the composition for 30 minutes. As shown by Table 8, Composition D was much more effective in removing the lipid component of human cerumen and the protein and lipid components of artificial cerumen than the five percent sodium bicarbonate solution.

In addition, it was unexpectedly discovered that composition D exhibits a synergistic effect in removing both human and artificial cerumen. Referring again to Table 8, the absorbency measurements for the bicarbonate component $D_1$ of Composition D, the proteolytic enzyme component $D_2$ of Composition D, and the vehicle $V_2$ of Composition D are also provided. As shown by Table 4, the sum of the absorbency measurements of the bicarbonate component $D_1$ and the proteolytic enzyme component $D_2$, subtracting any contribution from vehicle $V_2$, is significantly less than the absorbency measurement for Composition D, subtracting any contribution from vehicle $V_2$, for the protein component and the lipid component of both human and artificial cerumen.

## TABLE 8

### Efficacy Results
(Absorbency Units Per Gram Per ml at 30 Minutes)

| Cerumen | $D_1$ | $D_2$ | $D$ | 5% Sodium Bicarbonate Solution | $V_2$ | $D_1$-$V_2$ | $D_2$-$V_2$ | ($D_1$-$V_2$) + ($D_2$-$V_2$) | $D$-$V_2$ |
|---|---|---|---|---|---|---|---|---|---|
| Human 280 nm | 897 | 716 | 1153 | 1169 | 675 | + 222 | +41 | +263 | +478 |
| Human 600 nm | 12.2 | 2.9 | 14.0 | 8.8 | 3.0 | +9.2 | -0.1 | +9.1 | +11.0 |
| Artificial 280 nm | 557 | 49 | 812 | 577 | 31 | +526 | +18 | +544 | +781 |
| Artificial 280 nm | 40 | 1.2 | 49 | 39 | 0.6 | +39.4 | +0.6 | +40.0 | +48.4 |

EP 1 337 228 B1

## EXAMPLE 8

Table 9 shows the composition of the first part of Composition D before mixing with the second part of Composition D. Table 10 shows the composition of the second part of Composition D before mixing with the first part of Composition D. Composition D is preferably made by simple of the composition of Table 9 with the composition of Table 10. A ll ingredient amounts are in units of weight/volume percent unless otherwise indicated.

TABLE 9

| (First Part of Composition D) | |
|---|---|
| INGREDIENT | AMOUNT |
| Glycerin | 46.7 |
| Methyl Trypsin | 1335 AU/ml |
| Purified water | q.s. |

TABLE 10

| (Second Part of Composition D) | |
|---|---|
| INGREDIENT | AMOUNT |
| Sodium Bicarbonate | 5.88 |
| Benzalkonium Chloride | 0.012 |
| Hydrochloric Acid | q.s. to pH 8.0 |
| Purified water | q.s. |

Various volumes of the first part and the second part of Composition D may be employed. The preferred ratio of the volume of the first part of Composition D to the volume of the second part of Composition D is 3:17. A preferred volume of the first part is 1.5 ml. A preferred volume of the second part is 8.5 ml. The glycerin in the first part of Composition D acts as an enzyme stabilizing agent for the methyl trypsin. After the first part and the second part of Composition D are mixed, the glycerin also acts as a demulcent.

## EXAMPLE 9

Example 9 is another example of the third composition of present invention containing calcium chloride and boric acid as enzyme stabilizing agents. Table 11 shows the first part of the composition, which is preferably housed in upper container 14 of device 10. Table 12 shows the second part of the composition, which is preferably housed in lower container 12 of device 10. Table 13 shows the mixed composition, which is preferably made by simple mixing of the composition of Table 11 with the composition of Table 12 in the preferred volume ratio of 3:17. All ingredient amounts are in units of weight/volume percent unless otherwise indicated. The glycerin in the first part of the composition acts as an enzyme stabilizing agent for the methyl trypsin. After the first part and the second part of the composition are mixed, the glycerin also acts as a demulcent.

TABLE 11

| (First Part of Composition) | |
|---|---|
| INGREDIENT | AMOUNT |
| Glycerin | 46.7 |
| Methyl Trypsin | 1335 AU/ml |
| Boric Acid | 1.5 |
| Calcium Chloride | 0.25 |
| NaOH/HCl | Adjust to pH 7 |

TABLE 11 (continued)

| (First Part of Composition) | |
|---|---|
| **INGREDIENT** | **AMOUNT** |
| Purified water | q.s. |

TABLE 12

| (Second Part of Composition) | |
|---|---|
| **INGREDIENT** | **AMOUNT** |
| Tetronic®1304 | 0.294 |
| Sodium Bicarbonate | 5.882 |
| Benzalkonium Chloride | 0.012 |
| Sodium Citrate.2H$_2$O | 3.529 |
| Citric Acid | q.s. to pH 8.0 |
| Purified water | q.s. |

TABLE 13

| (Mixed Composition) | |
|---|---|
| **INGREDIENT** | **AMOUNT** |
| Methyl Trypsin | 200 AU/ml |
| Glycerin | 7.0 |
| Sodium Bicarbonate | 5.0 |
| Tetronic® 1304 | 0.25 |
| Boric Acid | 0.225 |
| Calcium Chloride | 0.0375 |
| Benzalkonium Chloride | 0.01 |
| Sodium Citrate.2H$_2$O | 3.0 |
| Citric acid | q.s. to pH 8.0 |
| Purified water | q.s. |

**EXAMPLE 10**

Table 14 shows another example of the composition of a first part of Composition C before mixing with the second part of Composition C. Table 15 shows another example of the composition of the second part of Composition C before mixing with the first part of Composition C. All ingredient amounts are in units of weight/volume percent unless otherwise indicated.

## TABLE 14
### (First Part of Composition C)

| INGREDIENT | AMOUNT |
|---|---|
| Methyl Trypsin | 2.22 mg (2000 AU) |
| Sodium Citrate.$2H_2O$ | 187.4 mg |
| Citric Acid (Anhydrous) | 4.2 mg |
| Glycerin | 2.0 mg |
| Tetronic® 1304 | 4.0 mg |
| Dehydrated Alcohol | 28 mg |

## TABLE 15
### (Second Part of Composition C)

| INGREDIENT | AMOUNT |
|---|---|
| Benzalkonium Chloride | 0.01 |
| Glycerin | 6.98 |
| Sodium Bicarbonate | 5.00 |
| Sodium Citrate.$2H_2O$ | 1.13 |
| Tetronic® 1304 | 0.21 |
| Citric Acid | q.s. pH 8.0 |
| Purified water | q.s. to 100 ml |

The first part of composition C is a powder or granulate. The composition of Example 10 is thus preferred when it is desired for the first part of the composition to be in powder or granulate form. Various masses and volumes of the first part and the second part of Composition C may be employed, respectively. The preferred mass of the first part is 200 mg, and the quantities in Table 14 are for 200 mg of the first part of Composition C. The preferred volume of the second part is 10 ml. 1 mg of methyl trypsin equals 900 AU. The dehydrated alcohol component of the first part is evaporated during manufacture. When the first part and second parts are mixed, the resulting Composition C has an identical formulation to Composition C shown in Table 1 of Example 1. The glycerin in the first part of Composition C acts as an enzyme stabilizing agent for the powder or granulate methyl trypsin, and as a binder between the sodium citrate.$2H_2O$ and anhydrous citric acid. The glycerin in the second part of Composition C, and in the composition after the first part and the second part of Composition C are mixed, also acts as a demulcent. The pH of the first part of Composition C is preferably about 6.5.

Composition C of Example 10 is preferably made using the following technique. The first part of Composition C, as shown in Table 14, is preferably prepared as follows. 187.4 mg of sodium citrate.$2H_2O$ is passed through a high-speed, rotary, 450 µm sieve into a planetary mixer. 2.0 mg of glycerin is then dissolved under agitation in 14 mg of dehydrated alcohol in a compounding vessel. The compounding vessel is preferably made of glass or stainless steel. The alcohol is preferably anhydrous ethanol. 4.2 mg of anhydrous citric acid is then added to the compounding vessel and allowed to dissolve under agitation. The contents of the compounding vessel are then added to the sodium citrate.$2H_2O$ in the planetary mixer, and the resulting mixture is mixed in the planetary mixer for 10 minutes. The mixture will form a granulate. The granulate fixed in the shovels of the planetary mixer is then removed, and the mixture is mixed in the planetary mixer for an additional 5 minutes. The wet granulate is removed from the planetary mixer, extended onto a tray, covered with a cloth, and allowed to dry at room temperature for at least 12 hours. The dried granulate is passed through a high speed, rotary, 450 μm sieve to form a preliminary granulate.

Methyl trypsin is milled using a Waring blender that is preferably rotating at 18,000 to 25,000 rpm, and most preferably at 21,000 rpm. Approximately 20 percent excess methyl trypsin (2.7 mg total) is used to compensate for losses during manufacture. Small quantities of the preliminary granulate and small quantities of the methyl trypsin are alternatively added to a clean planetary mixer and mixed until homogeneous. This mixing typically takes about 10 minutes. 4.0 mg of Tetronic® 1304 is then dissolved under agitation in 14 mg of dehydrated alcohol in a compounding vessel. The compounding vessel is preferably made of glass or stainless steel. The alcohol is preferably anhydrous ethanol. The Tetronic® 1304 and anhydrous ethanol are then added to the homogeneous mixture of preliminary granulate and methyl trypsin in the planetary mixer and are mixed for 5 minutes. The Tetronic acts as a binder for the methyl trypsin and the preliminary granulate. The granulate fixed in the shovels of the planetary mixer is then removed, and the mixture is mixed in the planetary mixer for an additional 5 minutes. The wet granulate is removed from the planetary mixer, extended onto a tray, covered with a cloth, and allowed to dry at room temperature for at least 12 hours. The dried granulate is passed through a high-speed, rotary, 450 μm sieve to form the final granulate or powder of the first part of Composition C. It has been discovered that the final granulate or powder of the first part of Composition C, when prepared using the above-described technique, has a stable concentration of methyl trypsin at up to 40 degrees Celsius for at least 3 months after preparation.

The second part of Composition C, as shown in Table 15, is preferably prepared as follows. The amounts in parenthesis represent the amounts for a 10 ml volume of the second part of Composition C. An amount of purified water equivalent to about 50% of the required volume (5.0 ml) is transferred to a suitable compounding vessel. 0.21 weight/volume percent (.021 g) of Tetronic® 1304 is then added to the compounding vessel and allowed to dissolve. The Tetronic® 1304 in the second part of Composition C, and in the composition after the first part and the second part of Composition C are mixed, also acts as a surfactant. Next, 5.00 weight/volume percent (0.5 g) of sodium bicarbonate is added to the compounding vessel under agitation at a low speed and allowed to dissolve. 6.98 weight/volume percent (0.698 g) of glycerin is then added to the compounding vessel under agitation and allowed to dissolve. Next, 0.01 weight/volume percent plus an additional 5% excess to compensate for loss during manufacture (0.0021 ml of a 50 weight/volume percent solution in water) of benzalkonium chloride is added to the compounding vessel under agitation and allowed to mix. 1.13 weight/volume percent sodium citrate.$2H_2O$ (0.113 g) is then added to the compounding vessel under agitation and allowed to dissolve. The pH of the mixture in the compounding vessel is then adjusted to 8.0 by adding citric acid. The citric acid is preferably anhydrous. Purified water is then added to the compounding vessel under agitation to reach the desired volume of the second part of Composition C of 10 ml. The final pH of the mixture is then checked. Next, the mixture is filtered through a 0.22 μm membrane to obtain the final second part of Composition C. Composition C is then made by simple mixing of the composition of Table 14 with the composition of Table 15.

From the above, it may be appreciated that the present invention provides improved compositions for assisting in the removal of human cerumen. In addition, the compositions of the present invention are useful for the treatment of other otic and nasal conditions. For example, given the presence of a tympanostomy tube or other rupture or hole in the tympanic membrane, the compositions of the present invention may be used to cleanse the middle and external ear of the viscous exudate that often results from a middle ear infection. This viscous exudate, which is commonly called "glue ear", includes conditions such as secretory otitis media, mucoid otitis media, serous otitis media, and chronic otitis media with effusion. These conditions sometimes lead to hearing loss via inflammation and/or the inhibition of ossicular sound conduction by the exudate. As another example, the compositions of the present invention may be applied to the inner tissues of the nose via irrigation or spray to dissolve nasal crusts or scabs that result from surgery or other insult or trauma to the nasal tissue. Such crusts and scabs are typically a mixture of blood, mucus, and secretions from open mucosal surfaces. As a further example, the compositions of the present invention may be used to dissolve other crusts or scabs.

The present invention has been described by reference to certain preferred embodiments. However, it should be understood that it may be embodied in other specific forms or variations thereof without departing from its spirit or essential characteristics. The embodiments described above are therefore considered to be illustrative in all respects and not restrictive, the scope of the invention being indicated by the appended claims rather than by the foregoing description.

**Claims**

**1.** A composition, comprising:

a cerumenolytically acceptable enzyme in an amount effective to assist in the removal of human cerumen from the external ear canal; a bicarbonate in an amount effective to assist in the removal of human cerumen from the external ear canal; and an aqueous otologically acceptable vehicle.

2. The composition of claim 1 wherein said cerumenolytically acceptable enzyme is selected from the group consisting of lipases, proteases, amylases, and combinations or mixtures thereof.

3. The composition of claim 2 wherein said cerumenolytically acceptable enzyme is a proteolytic enzyme.

4. The composition of claim 3 wherein said proteolytic enzyme is selected from the group consisting of pancreatin, trypsin, subtilisin, collagenase, keratinase, carboxypeptidase, papain, bromelain, aminopeptidase, elastase, Aspergillo peptidase, pronase E (from S. griseus), dispase (from Bacillus polymyxa), and combinations or mixtures thereof.

5. The composition of claim 3 wherein said proteolytic enzyme comprises a microbially derived enzyme.

6. The composition of claim 3 wherein said proteolytic enzyme comprises an alkyl trypsin.

7. The composition of claim 6 wherein said alkyl trypsin comprises methyl trypsin.

8. The composition of any of claims 1 to 7 wherein said vehicle comprises a demulcent.

9. The composition of claim 8 wherein said demulcent is selected from the group consisting of povidone, polyvinyl alcohol, glycerin, propylene glycol, polyethylene glycol, and cellulose derivatives.

10. The composition of claim 9 wherein said demulcent is glycerin.

11. The composition of any of claims 1 to 10 wherein said vehicle comprises a surfactant.

12. The composition of claim 11 wherein said surfactant is selected from the group consisting of polysorbates, 4-(1,1,3,3-tetramethylbutyl) phenol/poly(oxyethylene) polymers, poly(oxyethylene)-poly(oxypropylene) block copolymers, polyethylene glycol esters of fatty acids, and polyoxypropylene ethers of higher alkanes ($C_{12}$-$C_{18}$).

13. The composition of claim 12 wherein said surfactant is a poly(oxyethylene)-poly(oxypropylene) block copolymer.

14. The composition of any of claims 1 to 13 wherein said vehicle comprises a preservative.

15. The composition of claim 14 wherein said preservative is selected from the group consisting of poly[dimethylimino-2-butene-1,4-diyl]chloride-alpha-[4-tris(2-hydroxyethyl)-ammonium]dichloride, benzalkonium halides, alexidine salts, chlorhexidine salts, hexamethylene biguanimides and their polymers; and combinations or mixtures thereof.

16. The composition of claim 15 wherein said preservative is a benzalkonium halide.

17. The composition of any of claims 1 to 16 wherein said vehicle comprises a buffer.

18. The composition of claim 17 wherein said buffer is selected from the group consisting of citrate, phosphate, borate, acetate, Tris, salts of any of the foregoing, and combinations or mixtures thereof.

19. The composition of claim 18 wherein said buffer is a citrate or salt thereof.

20. The composition of any of claims 1 to 19 wherein said cerumenolytically acceptable enzyme is methyl trypsin, and said methyl trypsin is present in the amount of 50 AU/ml to 500 AU/ml.

21. The composition of any of claims 1 to 20 further comprising an enzyme stabilizing agent.

22. The composition of claim 21 wherein said enzyme stabilizing agent is selected from the group consisting of monomeric polyols, polymeric polyols, calcium ions, and borate/boric acid compound.

**23.** The composition of claim 22 wherein said enzyme stabilizing agent is glycerin.

**24.** The composition of any of claims 1 to 23 wherein said bicarbonate is sodium bicarbonate.

**25.** The composition of claim 24 wherein said sodium bicarbonate is present in the amount 0.5 weight/volume percent to 15 weight/volume percent.

**26.** The composition of claim 25 wherein said sodium bicarbonate is present in the amount of 5 weight/volume percent and said cerumenolytically acceptable enzyme is methyl trypsin which is present in the amount of 200 AU/ml.

**27.** The composition of any of claims 1 to 26 wherein the composition is packaged as two separate parts wherein the first part comprises a cerumenolytically acceptable enzyme in an amount effective to assist in the removal of human cerumen from the external ear canal; and the second part comprises an aqueous otologically acceptable vehicle.

**28.** The composition of claim 27 wherein the bicarbonate is present in the vehicle.

**29.** The composition of claim 27 or 28 wherein the demulcent is present in the vehicle.

**30.** The composition of any of claims 27 to 29 wherein the surfactant is present in the vehicle.

**31.** The composition of any of claims 27 to 30 wherein the preservative is present in the vehicle.

**32.** The composition of any of claims 27 to 31 wherein the buffer is present in the vehicle.

**33.** The composition of any of claims 27 to 32 wherein the enzyme stabilizing agent is present in the first part.

**34.** The composition of any of claims 27 to 33 wherein said first part and said second part are packaged in separate bottles.

**35.** The composition of any of claims 27 to 33 wherein said first part and said second part are packaged in a device having a first container receiving said first part, a second container receiving said second part, and a non-permeable membrane separating said first container from said second container, and wherein said non-permeable membrane may be tom to allow mixing of said first part and said second part.

**36.** Use of a composition as claimed in any of claims 1 to 35 for the preparation of a medicament for removing human cerumen.

**37.** Method of preparing a composition as claimed in any of claims 1 to 26 wherein a first composition comprising the cerumenolytically acceptable enzyme and optionally the enzyme stabilizer is mixed with a composition comprising the remainder of the otologically acceptable vehicle.

**38.** Method according to claim 37 wherein the bicarbonate is present in the composition comprising the remainder of the otologically acceptable vehicle.

**39.** Device for the storage of a composition as claimed in any of claims 1 to 26 wherein the device consists of two containers wherein the first container contains the cerumenolytically acceptable enzyme and optionally the enzyme stabilizer and the second container contains the remainder of the otologically acceptable vehicle.

**40.** Device according to claim 39 wherein the first container and the second container are separate bottles.

**41.** Device according to claim 39 wherein the first container and the second container are separated by a non-permeable membrane which can be tom to allow mixing of the composition of the first container with the composition of the second container.

**42.** Device according to claim 39 wherein the bicarbonate is present in the second container which contains the remainder of the otologically acceptable vehicle.

# EP 1 337 228 B1

**Patentansprüche**

1. Zusammensetzung enthaltend ein cerumenolytisch annehmbares Enzym in einer Menge, die wirksam ist, um die Entfernung von menschlichem Ohrenschmalz aus dem äußeren Ohrkanal zu fördern; ein Bicarbonat in einer Menge, die wirksam ist, um die Entfernung von menschlichem Ohrenschmalz aus dem äußeren Ohrkanal zu fördern, und einen wässrigen für das Ohr annehmbaren Träger.

2. Zusammensetzung nach Anspruch 1, wobei das berumenolytisch annehmbare Enzym ausgewählt ist aus der Gruppe bestehend aus Lipasen, Proteasen, Amylasen und Kombinationen oder Mischungen davon.

3. Zusammensetzung nach Anspruch 2, wobei das cerumenolytisch annehmbare Enzym ein proteolytisches Enzym ist.

4. Zusammensetzung nach Anspruch 3, wobei das proteolytische Enzym ausgewählt ist aus der Gruppe bestehend aus Pankreatin, Trypsin, Subtilisin, Collagenase, Keratinase, Carboxypeptidase, Papain, Bromelain, Aminopeptidase, Elastase, Aspergillus-Peptidase, Pronase E (aus S. griseus), Dispase (aus Bacillus polymyxa) und Kombinationen oder Mischungen davon.

5. Zusammensetzung nach Anspruch 3, wobei das proteolytische Enzym ein aus Mikroben stammendes Enzym enthält.

6. Zusammensetzung nach Anspruch 3, wobei das proteolytische Enzym ein Alkyltrypsin enthält.

7. Zusammensetzung nach Anspruch 6, wobei das Alkyltrypsin Methyltrypsin enthält.

8. Zusammensetzung nach einem der Ansprüche 1 bis 7, wobei der Träger ein Demulcens aufweist.

9. Zusammensetzung nach Anspruch 8, wobei das Demulcens ausgewählt ist aus der Gruppe bestehend aus Povidone, Polyvinylalkohol, Glycerin, Propylenglycol, Polyethylenglycol und Cellulosederivaten.

10. Zusammensetzung nach Anspruch 9, wobei das Demulcens Glycerin ist.

11. Zusammensetzung nach einem der Ansprüche 1 bis 10, wobei der Träger ein Tensid enthält.

12. Zusammensetzung nach Anspruch 11, wobei das Tensid ausgewählt ist aus der Gruppe bestehend aus Polysorbaten, 4-(1,1,3,3-Tetramethylbutyl)phenol/Poly(oxyethylen)polymeren, Poly(oxyethylen)-Poly-(oxypropylen)-Blockcopolymeren, Polyethylenglycolestern von Fettsäuren und Polyoxypropylenethern von höheren Alkanen ($C_{12}$-$C_{18}$).

13. Zusammensetzung nach Anspruch 12, wobei das Tensid ein Poly(oxyethylen)-Poly(oxypropylen)-Blockcopolymer ist.

14. Zusammensetzung nach einem der Ansprüche 1 bis 13, wobei der Träger ein Konservierungsmittel aufweist.

15. Zusammensetzung nach Anspruch 14, wobei das Konservierungsmittel ausgewählt ist aus der Gruppe bestehend aus Poly[dimethylimino-2-buten-1,4-diyl]chlorid-alpha-[4-tris-(2-hydroxyethyl)ammonium]-dichlorid, Benzalkoniumhalogeniden, Alexidinsalzen, Chlorhexidinsalzen, Hexamethylenbiguanimiden und deren Polymeren und Kombinationen oder Mischungen davon.

16. Zusammensetzung nach Anspruch 15, wobei das Konservierungsmittel ein Benzalkoniumhalogenid ist.

17. Zusammensetzung nach einem der Ansprüche 1 bis 16, wobei der Träger einen Puffer enthält.

18. Zusammensetzung nach Anspruch 17, wobei der Puffer ausgewählt ist aus der Gruppe bestehend aus Citrat, Phosphat, Borat, Acetat, Tris, Salzen von jedem der vorhergehenden und Kombinationen oder Mischungen davon.

19. Zusammensetzung nach Anspruch 18, wobei der Puffer ein Citrat oder Salz davon ist.

22

**20.** Zusammensetzung nach einem der Ansprüche 1 bis 19, wobei das cerumenolytisch annehmbare Enzym Methyltrypsin ist und das Methyltrypsin in einer Menge von 50 AU/ml bis 500 AU/ml vorhanden ist.

**21.** Zusammensetzung nach einem der Ansprüche 1 bis 20, weiter enthaltend ein Enzym stabilisierendes Mittel.

**22.** Zusammensetzung nach Anspruch 21, wobei das Enzym stabilisierende Mittel ausgewählt ist aus der Gruppe bestehend aus monomeren Polyolen, polymeren Polyolen, Calciumionen und Borat/Borsäureverbindungen.

**23.** Zusammensetzung nach Anspruch 22, wobei das Enzym stabilisierende Mittel Glycerin ist.

**24.** Zusammensetzung nach einem der Ansprüche 1 bis 23, wobei das Bicarbonat Natriumbicarbonat ist.

**25.** Zusammensetzung nach Anspruch 24, wobei das Natriumbicarbonat in einer Menge von 0,5 % (Gewicht/Volumen) bis 15 % (Gewicht/Volumen) vorhanden ist.

**26.** Zusammensetzung nach Anspruch 25, wobei das Natriumbicarbonat in einer Menge von 5 % (Gewicht/Volumen) vorhanden ist und das cerumenolytisch annehmbare Enzym Methyltrypsin ist, das in einer Menge von 200 AU/ml vorhanden ist.

**27.** Zusammensetzung nach einem der Ansprüche 1 bis 26, wobei die Zusammensetzung in Form von zwei getrennten Teilen verpackt ist, wobei der erste Teil ein cerumenolytisch annehmbares Enzym in einer Menge aufweist, die wirksam ist, um die Entfernung von menschlichem Ohrenschmalz aus dem äußeren Ohrkanal zu fördern, und der zweite Teil einen wässrigen für das Ohr annehmbaren Träger enthält.

**28.** Zusammensetzung nach Anspruch 27, wobei das Bicarbonat in dem Träger vorhanden ist.

**29.** Zusammensetzung nach Anspruch 27 oder 28, wobei das Demulcens in dem Träger vorhanden ist.

**30.** Zusammensetzung nach einem der Ansprüche 27 bis 29, wobei das Tensid im Träger vorhanden ist.

**31.** Zusammensetzung nach einem der Ansprüche 27 bis 30, wobei das Konservierungsmittel in dem Träger vorhanden ist.

**32.** Zusammensetzung nach einem der Ansprüche 27 bis 31, wobei der Puffer in dem Träger vorhanden ist.

**33.** Zusammensetzung nach einem der Ansprüche 27 bis 32, wobei das Enzym stabilisierende Mittel in dem ersten Teil vorhanden ist.

**34.** Zusammensetzung nach einem der Ansprüche 27 bis 33, wobei der erste Teil und der zweite Teil in getrennten Flaschen verpackt sind.

**35.** Zusammensetzung nach einem der Ansprüche 27 bis 33, wobei der erste Teil und der zweite Teil in einer Vorrichtung verpackt sind mit einem ersten Behälter, der den ersten Teil aufnimmt, einem zweiten Behälter, der den zweiten Teil aufnimmt, und einer nicht durchlässigen Membran, die den ersten Behälter von dem zweiten Behälter trennt, und wobei die nicht durchlässige Membran zerrissen werden kann, damit sich erster und zweiter Teil mischen können.

**36.** Verwendung einer Zusammensetzung, wie in einem der Ansprüche 1 bis 35 beansprucht, zur Herstellung eines Arzneimittels zur Entfernung von menschlichem Ohrenschmalz.

**37.** Verfahren zur Herstellung einer Zusammensetzung wie in einem der Ansprüche 1 bis 26 beansprucht, wobei eine erste Zusammensetzung mit dem cerumenolytisch annehmbaren Enzym und gegebenenfalls dem Enzymstabilisator mit einer Zusammensetzung vermischt wird, die den Rest des für das Ohr annehmbaren Trägers enthält.

**38.** Verfahren nach Anspruch 37, wobei das Bicarbonat in der Zusammensetzung vorhanden ist, die den Rest des für das Ohr annehmbaren Trägers enthält.

**39.** Vorrichtung zur Aufbewahrung einer Zusammensetzung, wie in einem der Ansprüche 1 bis 26 beansprucht, wobei die Vorrichtung aus zwei Behältern besteht, wobei der erste Behälter das cerumenolytisch annehmbare Enzym und gegebenenfalls den Enzymstabilisator enthält und der zweite Behälter den Rest des für das Ohr annehmbaren Trägers enthält.

**40.** Vorrichtung nach Anspruch 39, wobei der erste Behälter und der zweite Behälter getrennte Flaschen sind.

**41.** Vorrichtung nach Anspruch 39, wobei der erste Behälter und der zweite Behälter durch eine nicht durchlässige Membran getrennt sind, die zerrissen werden kann, damit sich die Zusammensetzung des ersten Behälters mit der Zusammensetzung des zweiten Behälters vermischen kann.

**42.** Vorrichtung nach Anspruch 39, wobei das Bicarbonat in dem zweiten Behälter vorhanden ist, der den Rest des für das Ohr annehmbaren Trägers enthält.

**Revendications**

**1.** Composition, comprenant:

une enzyme acceptable sur le plan céruménolytique en une quantité efficace pour aider à enlever le cérumen humain du conduit auditif externe ; un bicarbonate en une quantité efficace pour aider à enlever le cérumen humain du conduit auditif externe ; et un véhicule aqueux otologiquement acceptable.

**2.** Composition selon la revendication 1, dans laquelle ladite enzyme acceptable sur le plan céruménoiytique est choisie dans le groupe constitué par les lipases, les protéases, les amylases, et des combinaisons ou des mélanges de celles-ci.

**3.** Composition selon la revendication 2, dans laquelle ladite enzyme acceptable sur le plan céruménolytique est une enzyme protéolytique.

**4.** Composition selon la revendication 3, dans laquelle ladite enzyme protéolytique est choisie dans le groupe constitué par la pancréatine, la trypsine, la subtilisine, la collagénase, la kératinase, la carboxypeptidase, la papaïne, la bromélaïne, l'aminopeptidase, l'élastase, l'Aspergillo peptidase, la pronase E (de S. griseus), la dispase (de Bacillus polymyxa), et des combinaisons ou des mélanges de celles-ci.

**5.** Composition selon la revendication 3, dans laquelle ladite enzyme protéolytique comprend une enzyme d'origine microbienne.

**6.** Composition selon la revendication 3, dans laquelle ladite enzyme protéolytique comprend une alkyl trypsine.

**7.** Composition selon la revendication 6, dans laquelle ladite alkyl trypsine comprend la méthyl trypsine.

**8.** Composition selon l'une quelconque des revendications 1 à 7, dans laquelle ledit véhicule comprend un adoucissant.

**9.** Composition selon la revendication 8, dans laquelle ledit adoucissant est choisi dans le groupe constitué par la povidone, l'alcool polyvinylique, la glycérine, le propylèneglycol, le polyéthylèneglycol, et les dérivés Cellulosiques.

**10.** Composition selon la revendication 9, dans laquelle ledit adoucissant est la glycérine.

**11.** Composition selon l'une quelconque des revendications 1 à 10, dans laquelle ledit véhicule comprend un surfactant.

**12.** Composition selon la revendication 11, dans laquelle ledit surfactant est choisi dans le groupe constitué par les polysorbates, les polymères de 4-(1,1,3,3-tétraméthylbutyl)phénol/poly(oxyéthylène), les copolymères séquencés de poly(oxyéthylène)-poly(oxypropylène), les esters de polyéthylèneglycol d'acides gras, et les éthers de polyoxypropylène d'alcanes supérieurs ($C_{12}$-$C_{18}$).

**13.** Composition selon la revendication 12, dans laquelle ledit surfactant est un copolymère séquencé de poly(oxyéthylène)-poly(oxypropylène).

14. Composition selon l'une quelconque des revendications 1 à 13, dans laquelle ledit véhicule comprend un conservateur.

15. Composition selon la revendication 14, dans laquelle ledit conservateur est choisi dans le groupe constitué par le dichlorure de poly[diméthylimino-2-butène-1,4-diyl]chlorure-alpha-[4-tris(2-hydroxyéthyl)-ammonium], les halogénures de benzalkonium, les sels d'alexidine, les sels de chlorhexidine, les hexaméthylène biguanimides et leurs polymères ; et des combinaisons ou des mélanges de ceux-ci.

16. Composition selon la revendication 15, dans laquelle ledit conservateur est un halogénure de benzalkonium.

17. Composition selon l'une quelconque des revendications 1 à 16, dans laquelle ledit véhicule comprend un tampon.

18. Composition selon la revendication 17, dans laquelle ledit tampon est choisi dans le groupe constitué par le citrate, le phosphate, le borate, l'acétate, Tris, les sels de l'un quelconque de ceux-ci, et des combinaisons ou des mélanges de ceux-ci.

19. Composition selon la revendication 18, dans laquelle ledit tampon est un citrate ou un sel de celui-ci.

20. Composition selon l'une quelconque des revendications 1 à 19, dans laquelle ladite enzyme acceptable sur le plan céruménolytique est la méthyl trypsine, et ladite méthyl trypsine est présente en la quantité de 50 AU/mL à 500 AU/mL.

21. Composition selon l'une quelconque des revendications 1 à 20, comprenant en outre un agent stabilisant enzymatique.

22. Composition selon la revendication 21, dans laquelle ledit agent stabilisant enzymatique est choisi dans le groupe constitué par les polyols monomères, les polyols polymères, les ions calcium, et un composé borate/acide borique.

23. Composition selon la revendication 22, dans laquelle ledit agent stabilisant enzymatique est la glycérine.

24. Composition selon l'une quelconque des revendications 1 à 23, dans laquelle ledit bicarbonate est le bicarbonate de sodium.

25. Composition selon la revendication 24, dans laquelle ledit bicarbonate de sodium est présent en la quantité de 0,5 % poids/volume à 15 % poids/volume.

26. Composition selon la revendication 25, dans laquelle ledit bicarbonate de sodium est présent en la quantité de 5 % poids/volume et ladite enzyme acceptable sur le plan céruménolytique est la méthyl trypsine qui est présente en la quantité dé 200 AU/mL.

27. Composition selon l'une quelconque des revendications 1 à 26, dans laquelle la composition est emballée sous la forme de deux parties séparées dans lesquelles la première partie comprend une enzyme acceptable sur le plan céruménolytique en une quantité efficace pour aider à enlever le cérumen humain du conduit auditif externe ; et la seconde partie comprend un véhicule aqueux otologiquement acceptable.

28. Composition selon la revendication 27, dans laquelle le bicarbonate est présent dans le véhicule.

29. Composition selon la revendication 27 ou 28, dans laquelle l'adoucissant est présent dans le véhicule.

30. Composition selon l'une quelconque des revendications 27 à 29, dans laquelle le surfactant est présent dans le véhicule.

31. Composition selon l'une quelconque des revendications 27 à 30, dans laquelle le conservateur est présent dans le véhicule.

32. Composition selon l'une quelconque des revendications 27 à 31, dans laquelle le tampon est présent dans le véhicule.

33. Composition selon l'une quelconque des revendications 27 à 32, dans laquelle l'agent stabilisant enzymatique est présent dans la première partie.

34. Composition selon l'une quelconque des revendications 27 à 33, dans laquelle ladite première partie et ladite seconde partie sont emballées dans des bouteilles séparées.

35. Composition selon l'une quelconque des revendications 27 à 33, dans laquelle ladite première partie et ladite seconde partie sont emballées dans un dispositif ayant un premier conteneur recevant ladite première partie, un second conteneur recevant ladite seconde partie, et une membrane non perméable séparant ledit premier conteneur dudit second conteneur, et dans laquelle ladite membrane non perméable peut être rompue pour permettre le mélange de ladite première partie et de ladite seconde partie.

36. Utilisation d'une composition selon l'une quelconque des revendications 1 à 35, pour la préparation d'un médicament destiné à enlever le cérumen humain.

37. Procédé de préparation d'une composition selon l'une quelconque des revendications 1 à 26, dans lequel une première composition comprenant l'enzyme acceptable sur le' plan cérumenolytique et facultativement le stabilisant enzymatique est mélangée avec une composition comprenant le reste du véhicule otologiquement acceptable.

38. Procédé selon la revendication 37, dans lequel le bicarbonate est présent dans la composition comprenant le reste du véhicule otologiquement acceptable.

39. Dispositif pour le stockage d'une composition selon l'une quelconque des revendications 1 à 26, dans lequel le dispositif est constitué par deux conteneurs dans lesquels le premier conteneur contient l'enzyme acceptable sur le plan cérumenolytique et facultativement le stabilisant enzymatique et le second conteneur contient le reste du véhicule otologiquement acceptable.

40. Dispositif selon la revendication 39, dans lequel le premier conteneur et le second conteneur sont des bouteilles séparées.

41. Dispositif selon la revendication 39, dans lequel le premier conteneur et le second conteneur sont séparés par une membrane non perméable qui peut être rompue pour permettre le mélange de la composition du premier conteneur avec la composition du second conteneur.

42. Dispositif selon la revendication 39, dans lequel le bicarbonate est présent dans le second conteneur qui contient le reste du véhicule otologiquement acceptable.

FIG. 1

FIG. 2

FIG. 3    FIG. 4

FIG. 5

FIG. 6

FIG. 7

FIG. 8

FIG. 9

FIG. 10